# EUROPÄISCHE PATENTSCHRIFT

(11) **EP 1 512 682 B1**
(45) Veröffentlichungstag und Bekanntmachung des Hinweises auf die Patenterteilung: **27.08.2008**
(21) Anmeldenummer: 04103133.7
(22) Anmeldetag: 02.07.2004
(51) Int. Cl.: C07C 263/04, C07C 263/06, C07C 265/14

(54) **Mehrstufiges Verfahren zur kontinuierlichen Herstellung von cycloaliphatischen Diisocyanaten**
Multi-step process for the continuous preparation of cycloaliphatic diisocyanates
Procédé à plusieurs étapes pour la préparation continue de diisocyanates cycloaliphatiques

(30) Priorität: 22.08.2003 DE 10338509
(43) Veröffentlichungstag der Anmeldung: 09.03.2005
(73) Patentinhaber: Evonik Degussa GmbH, 45128 Essen (DE)
(72) Erfinder: Kohlstruk, Stephan, Dr., 48249, Dülmen (DE); Kreczinski, Manfred, 44652, Herne (DE); Elm, Rainer, Dr., 45772, Marl (DE); Michalczak, Hans-Werner, 44651, Herne (DE)

(56) Entgegenhaltungen:
- EP-A- 0 568 782

## Beschreibung

Die Erfindung betrifft ein mehrstufiges Verfahren zur kontinuierlichen und phosgenfreien Herstellung von cycloaliphatischen Diisocyanaten.

Der synthetische Zugang zu Isocyanaten kann über eine Reihe unterschiedlicher Routen erfolgen. Älteste und auch heute noch vorherrschende Variante zur großtechnischen Herstellung von Isocyanaten ist die so genannte Phosgenroute. Grundlage dieses Verfahrens ist Umsetzung von Aminen mit Phosgen. Nachteil des Phosgenverfahrens ist der Einsatz von Phosgen, dass aufgrund seiner Toxizität und Korrosivität besonders hohe Anforderungen an seine Handhabung im industriellen Maßstab stellt.

Es gibt mehrere Verfahren, die Verwendung von Phosgen zur Herstellung von Isocyanaten in technischen Größenordnungen zu umgehen. Der Begriff phosgenfreies Verfahren wird häufig im Zusammenhang mit der Überführung von Aminen in Isocyanate unter Einsatz alternativer Carbonylierungsmittel, z.B. Harnstoff oder Dialkylcarbonat, benutzt (EP 0 018 586, EP 0 355 443, US 4 268 683, EP 0 990 644).

Grundlage der so genannten Harnstoffroute ist die Harnstoff-vermittelte Überführung von Diaminen in Diisocyanate über einen zweistufigen Prozess. Im ersten Schritt wird ein Diamin mit Alkohol in Gegenwart von Harnstoff oder Harnstoff-Äquivalenten (z. B. Alkylcarbonate, Alkylcarbamate) zu einem Diurethan umgesetzt, welches üblicherweise eine Zwischenreinigungstufe durchläuft und dann im zweiten Schritt thermisch in Diisocyanat und Alkohol gespalten wird (EP 0 355 443, US 4,713,476, US 5,386,053). Alternativ kann der eigentlichen Urethanbildung auch die separate Herstellung eines Diharnstoffs durch gezielte Umsetzung des Diamins mit Harnstoff vorgeschaltet sein (EP 0 568 782). Denkbar ist auch eine zweistufige Sequenz aus partieller Umsetzung von Harnstoff mit Alkohol im ersten und anschließender Zudosierung und Urethansierung des Diamins im zweiten Schritt (EP 0 657 420).

Die thermische Spaltung von Urethanen in die entsprechenden Isocyanate und Alkohole ist seit langem bekannt und kann sowohl in der Gasphase bei hohen Temperaturen als auch bei relativ niedrigen Temperaturen in der Flüssigphase durchgeführt werden. Problematisch ist jedoch bei beiden Verfahrensweisen, dass durch die thermische Belastung grundsätzlich auch unerwünschte Nebenreaktionen stattfinden, die zum einen die Ausbeute mindern und zum anderen zur Bildung verharzender Nebenprodukte führen, die den Ablauf eines technischen Prozesses durch Belegungen und Verstopfungen in Reaktoren und Aufarbeitungsvorrichtungen erheblich stören.

Es hat daher nicht an Vorschlägen gefehlt, durch chemische und verfahrenstechnische Maßnahmen Ausbeuteverbesserungen zu erzielen und die unerwünschte Nebenproduktbildung einzuschränken. So wird in einer Reihe von Dokumenten der Einsatz von Katalysatoren beschrieben, die die Spaltreaktion der Urethane beschleunigen (DE 10 22 222, US 3,919,279, DE 26 35 490). Tatsächlich gelingt es in Gegenwart geeigneter Katalysatoren - hierbei handelt es sich um eine Vielzahl basischer, saurer sowie metallorgansicher Verbindungen - durchaus, die Isocyanatausbeute im Vergleich zur unkatalysierten Variante zu steigern. Die Bildung unerwünschter Nebenprodukte kann jedoch auch durch die Anwesenheit eines Katalysators nicht vermieden werden. Dasselbe gilt für die zusätzliche Verwendung von inerten Lösemitteln, wie sie ebenfalls in der US 3,919,279 und DE26 35 490 empfohlen werden, um eine gleichmäßige Verteilung der zugeführten Wärme und des Katalysators im Reaktionsmedium zu gewährleisten. Grundsätzlich hat die Verwendung von unter Rückfluss siedenden Lösemitteln jedoch eine Reduzierung der Raum/Zeit-Ausbeute an Isocyanaten zur Folge und ist darüber hinaus mit dem Nachteil eines zusätzlichen hohen Energieaufwands behaftet.

In der EP 0 054 817 angeführte Beispiele zur thermisch geführten katalysierten Spaltung von Monourethanen beschreiben die Teilausschleusung des Reaktionsgemisches zur Abtrennung der im Zuge der Urethanspaltung entstehenden verharzenden Nebenprodukte. Diese Prozedur dient der Vermeidung von Belegungen und Verstopfungen in Reaktoren und Aufarbeitungseinrichtungen. Hinweise, die auf eine Ausbeute steigernde Verwertung der Teilausschleusung hindeuten, gibt es nicht. Die EP 0 061 013 beschreibt einen ähnlichen Lösungsansatz, wobei die Thermolyse in diesem Fall in Gegenwart von Lösemitteln durchgeführt wird, deren Aufgabe offenbar in einer besseren Aufnahme der schwerflüchtigen Nebenprodukten besteht. Auch hier wird die Teilausschleusung nicht im Sinne einer Ausbeuteoptimierung verwertet.

Aus der EP 0 355 443 ist nun bekannt, dass eine Ausbeutesteigerung erzielt werden kann, wenn die während der Spaltung von Diurethanen im Spaltreaktor entstehenden höhermolekularen, verwertbaren und nicht verwertbaren Nebenprodukte zur Gewährleistung einer störungsfreien und selektiven Reaktion möglichst kontinuierlich aus dem Reaktor ausgeschleust werden und anschließend in Gegenwart von Alkohol zum großen Teil umgesetzt und dann in die Diurethanherstellung zurückgeführt werden. Die beschriebene Verfahrensweise ist mit einem hohen Energieaufwand verbunden, da die Abtrennung nicht verwertbarer Nebenprodukte aus dem Austrag der Diurethanherstellung destillativ erfolgt, wobei das gesamte Diurethan verdampft werden muss. Im Unterschied zur EP 0 355 443 wird der Urethanisierungsaustrag beim Verfahren der EP 0 566 925 in zwei Teilströme aufgeteilt, von denen nur einer destillativ von seinen hochsiedenden, nicht verwertbaren Nebenprodukten befreit wird, bevor die vereinigten Diurethanströme der Deblockierungsreaktion im Spaltreaktor zugeführt werden. Zudem wird die kontinuierliche Spaltreaktorausschleusung bei der EP 0 566 925 direkt, d.h. ohne Reurethanisierungsschritt, in die Diurethansynthese zurückgeführt.

Die Herstellung der Diurethane in einer Eintopfreaktion aus Harnstoff, Diamin und Alkohol unter gleichzeitiger Abtrennung von Ammoniak ist gängige Praxis und wird in einer Reihe von Patenschriften beschrieben (EP 0 018 568, EP 0 355 443, EP 0 566 925). Nachteilig ist, dass durch die simultane Umsetzung von Harnstoff, Alkohol und Diamin zwangsläufig und in größerer Menge Nebenprodukte gebildet werden, die die Selektivität der Umsetzung beeinträchtigen und die vor der thermischen Deblockierung der Diurethane abgetrennt werden müssen. Die EP 0 568 782 beansprucht daher ein kontinuierliches Verfahren zur Herstellung von (cyclo)aliphatischen Diisocyanaten, welches im wesentlichen drei Hauptschritte umfasst, von denen der erste die Bildung von Bisharnstoffen, der zweite die Bildung von Diurethanen aus den Bisharnstoffen und der dritte die Spaltung der Diurethane in flüssiger Phase zu den gewünschten Diisocyanaten beschreibt - d.h., die Herstellung des Diurethans erfolgt in zwei getrennten Stufen. Nach der Lehre der EP 0 568 782 wird der Austrag der Reaktionssequenz aus Bisharnstoff-Bildung und anschließender Diurethansynthese zunächst destillativ von Leicht- und Mittelsiedern wie Alkoholen, Carbamaten und Carbonaten befreit und der Hochsieder im Diurethan danach durch Kurzwegverdampfung abgetrennt. Das Diurethan wird thermisch deblockiert und ein Teil des Spaltsumpfes wird kontinuierlich ausgeschleust, mit Alkohol reurethanisiert und wieder in die Diurethansynthesestufe zurückgefährt. Nachteil dieser Vorgehensweise ist zum einen, dass der Druckdestillationsreaktor groß genug ausgelegt sein muss, die kombinierten Ströme aus der Bisharnstoff-Synthese und der Reurethanisierung bewältigen zu können. Proportional zur Reaktorgröße wachsen aber auch die notwendigen Investitionskosten. Zum anderen erschwert die kontinuierliche Rückführung des Reurethanisats in den Diurethanreaktor die Einstellung einer definierten und für die Diurethanherstellung optimalen Stöchiometrie, weil die Zusammensetzung des Reurethanisats in Abhängigkeit von den Betriebsbedingungen variiert. Dem Gesamtprozess wird auf diesem Wege Ausbeutepotential entzogen. Insbesondere wurde dies unter den Bedingungen des Standes der Technik bei der Herstellung cycloaliphatischer Di- oder Polyisocyanate beobachtet. Unter cycloaliphatisch ist definitionsgemäß zu verstehen, dass die Isocyanatgruppe direkt an einem cycloaliphatischen Kohlenwasserstoffrest (e. g. Cyclohexan) gebunden ist.

Zusammenfassend kann festgestellt werden, dass gemäß dem Stand der Technik die Herstellung von Di- und Polyisocyanaten bekannt ist, durch Reaktion entsprechender Ausgangsverbindung zu Urethanen (einstufig oder mehrstufig) und anschließende Aufarbeitung des erhaltenen Reaktionsgemisches, welches die Urethane enthält, zu einer Urethan-Fraktion, anschließender Spaltung der Urethane zu den entsprechenden Di- oder Polyisocyanaten und Reinisolierung dieses Verfahrensprodukts.

Der Erfindung lag die Aufgabe zugrunde, ein verbessertes Verfahren zur Herstellung von cycloaliphatischen Diisocyanaten bereitzustellen, das die oben genannten Nachteile vermeidet.

Gelöst wurde die Aufgabe durch ein mehrstufiges und kontinuierliches Verfahren, wobei die Bildung der Diurethane zweistufig ausgeführt ist, das von Leicht-, Mittel- und Hochsiedern befreite Diurethan unter Freisetzung des gewünschten Diioscyanats thermisch gespalten wird, ein Teil des Spaltsumpfes der Spaltapparatur kontinuierlich ausgeschleust und mit Alkohol reurethanisiert wird und die Rückführung des Reurethanisatstroms nicht in die Diurethanherstellung sondern direkt in die Leichtsiederabtrennung erfolgt.

Gegenstand der Erfindung ist mehrstufiges Verfahren zur kontinuierlichen Herstellung von cycloaliphatischen Diisocyanaten, durch Umsetzung von cycloalipahtischen Diaminen mit Kohlensäurederivaten und Alkoholen zu cycloaliphatischen Diurethanen und anschließenden thermischen Spaltung der Diurethane zu cycloaliphatischen Diisocyanten, dadurch gekennzeichnet, dass die Bildung der Diurethane zweistufig ausgeführt ist, das von Leicht-, Mittel- und Hochsiedern befreite Diurethan unter Freisetzung des gewünschten Diioscyanats thermisch gespalten wird, ein Teil des Spaltsumpfes der Spaltapparatur kontiniuerlich ausgeschleust und mit Alkohol reurethanisiert wird und die Rückführung des Reurethanisatstroms direkt in die Leichtsiederabtrennung erfolgt.

Gegenstand der Erfindung ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Kohlensäurederivaten und Alkoholen zu Diurethanen, und deren thermische Spaltung, wobei
a) cycloaliphatische Diamine der Formel (II)

   H₂N-R-NH₂

   wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)

   R¹-OH

   wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Katalysatoren zu Cycloalkylenbisharnstoffen der Formel (IV)

   H₂N-OC-HN-R-NH-CO-NH₂

   wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;
b) der anfallende Roh-Cycloalkylenbishamstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)

   R¹O-OC-HN-R-NH-CO-OR¹

   überführt wird;
c) aus der erhaltenen Reaktionsmischung der Alkohol, die Dialkylcarbonate und/oder Carbamidsäurealkylester abgetrennt und der Alkohol in die Reaktionsstufe a) zurückfährt wird;
d) der Stoffstrom aus Stufe c) destillativ in einen Wertstoffstrom und einen Nebenproduktstrom, der ausgeschleust wird, getrennt wird,
e) die über die Schritte c) und d) aufgereinigte Diurethane enthaltende Reaktionsmischung in Gegenwart eines Katalysators lösemittelfrei bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig ausgeschleust wird;
f) die Spaltprodukte durch Rektifikation in ein rohes Diisocyanat und Alkohol getrennt werden;
g) das rohe cycloaliphatische Diisocyanat durch Destillation gereinigt und die Reinproduktfraktion isoliert wird;
h) die Sumpfausschleusung aus e) mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;
i) optional die Reurethanisierungsreaktion h) in Gegenwart spezieller Katalysatoren, ausgewählt aus den Halogeniden von Fe(III) und/oder Cu(I), durchgeführt wird;
j) ein Teil der Sumpffraktion der Reindestillation g) kontinuierlich ausgeschleust und in die Spaltreaktion e), und/oder in die Urethanisierungsstufe h) geführt wird;
k) optional die bei der Reindestillation des rohen cycloaliphatischen Diisocyanats anfallende Kopffraktion ebenfalls in die Urethanisierungsstufe h) zurückgeführt wird;
l) der Reurethanisatstrom aus h) in Stufe c) zurückgeführt wird.

Nach dem erfindungsgemäßen Verfahren können cycloaliphatische Diisocyanate im kontinuierlichen Betrieb problemlos mit sehr guter Ausbeute hergestellt werden. Durch den Verzicht der Rückführung des in seiner Zusammensetzung variablen Reurethanisatstroms in die Diurethanherstellung resultieren zwei Vorteile für das erfindungsgemäße Mehrstufenverfahren: Zum einen wird der Diurethanreaktor mit einem im Vergleich zum Stand der Technik geringem Massenstrom belastet, so dass durch eine kleinere Auslegung des Reaktors Kosteneinsparungspotentiale gehoben werden können. Zum anderem ist gewährleistet, dass die Diurethansynthese jederzeit unter defmierten und im Sinne der Ausbeute optimierten stöchiometrischen Verhältnissen durchgeführt werden kann.
a) Zur Herstellung der Cycloalkylenbisharnstoffe der Formel (IV) in der Reaktionsstufe a) werden die cycloaliphatischen Diamine der Formel (II), mit Harnstoff in Gegenwart eines Alkohols der Formel (III), gegebenenfalls auch Mischungen solcher Alkohole, in einem Reaktor bei 100 bis 145 °C und einem Druck von 0,7 bis 1,8 bar umgesetzt, wobei das gebildete Ammoniak kontinuierlich ausgetrieben wird. Die Umsetzung erfolgt bevorzugt in einem Destillationsreaktor, wobei die Edukte in einem Molverhältnis von Diamin : Harnstoff : Alkohol von 1 : 2,0 bis 2,4: 3 bis 10 kontinuierlich auf den obersten Boden aufgegeben werden und das freigesetzte Ammoniak durch Alkoholbrüden, die im Sumpf des Destillationsreaktor eingeführt werden, ausgetrieben wird. Die erforderliche Verweilzeit beträgt 4 bis 10 Stunden, vorzugsweise 5 bis 9 Stunden. Die zum Austreiben des Ammoniaks im Sumpf eingebrachte Alkoholmenge beträgt 0,05 bis 3 kg/kg, vorzugsweise 0,1 bis 1 kg/kg Bisharnstoff, wobei die so eingetragene Alkoholmenge zusammen mit dem gebildeten Ammoniak am Kopf abgezogen, nach partieller Kondensation in einer Alkoholrückgewinnungskolonne vom restlichen Ammoniak befreit und in den Sumpf zurückgeführt wird.
b) Der im Sumpf des Destillationsreaktors anfallende, in Alkohol gelöste rohe Cycloalkylenbisharnstoff wird kontinuierlich in einen zweiten Reaktor gefahren, in dem die Umsetzung zum Diurethan bei erhöhter Temperatur und erhöhtem Druck erfolgt, wobei wiederum Ammoniak freigesetzt wird, welches aus Gründen des chemischen Gleichgewichts aus dem Reaktionsgemisch entfernt werden muss. Die weitere Umsetzung des rohen Cycloalkylenharnstoffs aus a) erfolgt vorzugsweise in einem Druckdestillationsreaktor und bei einem molaren Verhältnis von Bisharnstoff zu Alkohol von 1 : 5 bis 12. Dabei wird der Stoffstrom aus a) vorzugsweise kontinuierlich auf den obersten Boden des Druckdestillationsreaktors gefahren. Die Umsetzung findet in Abwesenheit oder Gegenwart von Katalysatoren bei Reaktionstemperaturen von 140 bis 270 °C, vorzugsweise 160 bis 250 °C und unter einem Druck, der 5 bis 20 bar, vorzugsweise 7 bis 15 bar beträgt, innerhalb von 2 bis 20 Stunden, vorzugsweise 8 bis 15 Stunden, statt. Die kontinuierlich Austreibung des freigesetzten Ammoniaks wird unterstützt durch Alkoholbrüden, die im Sumpf des Druckdestillationsreaktors eingebracht und zweckmäßigerweise in einem am Sumpf der Kolonnne angebrachten Verdampfer erzeugt werden.
   Zur Erhöhung der Reaktionsgeschwindigkeit können die Diurethane in Gegenwart von Katalysatoren hergestellt werden. Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14^{th} Edition, publiziert von Chemical Rubber Publishing Co. 2310 Superior Ave. N.E. Cleveland, Ohio, enthalten, beispielsweise Halogenide wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocaramate. Beispielhaft genannt seinen die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Kobalt und Nickel. Als typische Katalysatoren seinen beispielhaft folgende Verbindungen genannt: Lithiumethanolat, Lithiumbutanolat, Natriummethanolat, Kalium-tert.butanolat, butanolat, Magnesiumethanolat, Calciummethanolat, Zinn-(II)-chlorid, Zinn-(IV)-chlorid, Bleiacetat, Aluminiumtrichlorid, Bismuttrichlorid, Kupfer-(II)-acetat, Kupfer-(II)-chlorid, Zinkchlorid, Zinkoctoat, Titantetrabutanolat, Vanadiumtrichlorid, Vanadiumacetonylacetat, Mangan-(II)-acetat, Eisen-(II)-acetat, Eisen-(III)-acetat, Eisenoxalat, Cobaltchlorid, Cobaltnaphthenat, Nickelchlorid, Nickelnaphthenat sowie deren Mischungen. Die Katalysatoren können gegebenenfalls auch in Form ihrer Hydrate oder Ammoniakate zu Einsatz kommen.
   Ausgangsverbindungen für das erfindungsgemäße Verfahren sind Diamine der bereits obengenannten Formel (II), Alkohole der bereits obengenannten Formel (III) sowie Harnstoff. Geeignete Diamine der Formel (II) sind beispielsweise 1,4-Diaininocyclohexan, 4,4'-Methylendicyclohexyldiamin, 2,4-Methylendicyclohexyldiamin, 2,2'-Methylendicyclohexyldiamin und isomere cycloaliphatische Diamine sowie perhydriertes Methylendiphenyldiamin. Methylendiphenyldiamin (MDA) fällt herstellungsbedingt als Isomerenmischung aus 4,4'-, 2,4- und 2,2'-MDA an (s. z. B. DE 101 27 273). Perhydriertes Methylendiphenyldiamin wird durch vollständige Hydrierung von MDA erhalten und ist demzufolge eine Mischung aus isomeren Methylendicyclohexyldiaminen (H₁₂MDA), nämlich 4,4'-, 2,4- und 2,2'-H₁₂MDA. Bevorzugt werden als Diamine der Formel (II) 4,4'-Methylendicyclohexyldiamin, 2,4-Methylendicyclohexyldiamin und 2,2'-Methylendicyclohexyl-diamin sowie auch beliebige Mischungen mindestens zweier dieser Isomere eingesetzt. Selbstverständlich können auch Diamine zum Einsatz gelangen, die von der Formel (II) abweichen. Beispielhaft seinen 1,3-und 1,4-Diaminomethylcyclohexan und 3-Aminomethyl-3,5,5-trimethylcyclohexyl-amin aufgeführt. Der Einsatz von Aminen, die von der Formel (II) abweichen, ist jedoch nicht bevorzugt.
   Als Alkohole der Formel (III) eignen sich beliebige aliphatische oder cycloaliphatische Alkohole, die unter Normaldruck einen unterhalb 190 °C liegenden Siedepunkt aufweisen. Beispielhaft genannt seinen C1-C6-Alkanole wie z. B. Methanol, Ethanol, 1-Propanol, 1-Butanol, 2-Butanol, 1-Hexanol oder Cyclohexanol. Bevorzugt wird 1-Butanol als Alkohol verwendet.
   Im Zuge der Umsetzung des Reaktionsgemisches wird Ammoniak freigesetzt, dessen Entfernung aus dem Reaktionsgleichgewicht sich als vorteilhaft erwiesen hat. Beim Austrag des Ammoniaks aus dem Reaktor ist darauf zu achten, dass die Wandtemperaturen des Reaktors und des Austragsrohres oberhalb von 60 °C liegen, damit eine Belegung durch Ammoniumcarbaminat, welches in minimalen Mengen aus Ammoniak und Kohlendioxid durch Zersetzung von Harnstoff gebildet wird, vermieden werden kann. Es hat sich beispielsweise bewährt, die Umsetzung in einem Druckdestillationsreaktor durchzuführen, wobei das Reaktionsgemisch im Gegenstrom zu im Sumpf eingebrachten Alkoholbrüden gefährt wird und auf diese Weise eine derartig intensive Durchmischung der Flüssigkeit auf den Böden, die praktisch jeweils einer Kaskadenstufe entsprechen, erfolgt. Das am Kopf abgezogene, dampfförmige Gemisch aus Alkohol und Ammoniak kann, vorzugsweise unter dem Druck des Druckdestillationsreaktors und ohne es vorher zu kondensieren, in eine Destillationskolonne gefährt werden, um Ammoniak freien Alkohol zu gewinnen, der in den Sumpf des Druckdestillationsreaktors und der Kolonne zurückgeführt wird. Um eine Belegung des Rückflusskondensators mit Ammoniumcarbaminat zu verhindern, lässt man in diesem zur Einstellung der Temperatur am Kopf auf mindestens 60 °C einen entsprechenden Anteil an Alkohol zu.
c) Der überschüssige Alkohol, die Dialkylcarbonate, sofern solche gebildet wurden, oder Carbamidsäurealkylester oder Mischungen aus mindestens zwei dieser Komponenten werden einstufig, oder vorteilhafterweise zweistufig abgetrennt. Auf der ersten Stufe wird die Reaktionsmischung vom Druckniveau der Reaktionsstufe b) auf einen Druck von 1 bis 500 mbar, vorzugsweise 2 bis 150 mbar, entspannt und auf diese Weise in gasförmige Brüden, die die überwiegende Alkoholmenge sowie gegebenenfalls Dialkylcarbonate und/oder Carbamidsäurealkylester enthalten, und in einen flüssigen Austrag aufgetrennt. Im zweiten Schritt wird der flüssige Austrag durch Dünnschichtverdampfung bei 180 bis 250 °C, vorzugsweise 200 bis 230 °C, und einem Druck von 0,1 bis 20 mbar, vorzugsweise 1 bis 10 mbar, von gegebenenfalls vorhandenem restlichen Butanol sowie Mittelsiedern wie Dialkylcarbonaten und/oder Carbamidsäurealkylestern befreit, so dass der Rückstand im wesentlichen aus dem monomeren Diurethan und gegebenenfalls hochsiedenden Oligomeren besteht. Die Brüden können nach weiterer destillativer Reinigung in die Reaktionsstufe a) zurückgeführt werden.
d) Der nach Abtrennung der Brüden aus Stufe c) erhaltene flüssige, die monomeren Diurethane, und gegebenenfalls hochsiedende Oligomere enthaltende Stoffstrom wird destillativ, vorzugsweise mit Hilfe eines Dünnschicht- oder Kurzwegverdampfers, bei einer Temperatur von 180 bis 260 °C, vorzugsweise 200 bis 240 °C und unter einem Druck von 0,01 bis 10 mbar, vorzugsweise 0,02 bis 5 mbar in einen Wertstoffstrom, der die monomeren Diurethane und die leichter siedenden Nebenprodukte enthält, und einen nicht destillierbaren Nebenproduktstrom getrennt, der aus dem Herstellverfahren ausgeschleust und üblicherweise als stofflich nicht verwertbarer Rückstand verworfen wird.
   Optional kann der gegebenenfalls hochsiedende Oligomere enthaltende Stoffstrom aus Stufe c) vor seiner oben beschriebenen destillativen Aufreinigung auch in zwei Teilströme aufgeteilt werden, von denen einer direkt der Deblockierungsreaktion (siehe e)) zugeführt wird und der andere zunächst die soeben beschriebene Hochsiederabtrennung durchläuft.
e) Der die monomeren Diurethane und die leichter siedenden Nebenprodukte enthaltende Wertstoffstrom wird in einer geeigneten Vorrichtung teilweise, lösemittelfrei in flüssiger Phase in Gegenwart von Katalysatoren bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch gespalten. Der Umsatz von Diurethan zu Diisocyanat in der Vorrichtung zur thermischen Spaltung kann in Abhängigkeit vom verwendeten Diurethan weitgehend frei gewählt werden und liegt üblicherweise in einem Bereich von 10 bis 95 Gew.-%, vorzugsweise 35 bis 85 Gew.-% der zugeführten Diurethanmenge (Feed). Der ungespaltende Anteil der Reaktionsmischung, der nicht umgesetzte Diurethane, hochsiedende Nebenprodukte und andere wieder verwertbare und unverwertbare Nebenprodukte enthält, wird kontinuierlich ausgeschleust. Die Menge der Ausschleusung richtet sich u.a. nach dem gewünschten Umsatz und der gewünschten Kapazität der Spaltreaktion und kann leicht experimentell ermittelt werden. Sie beträgt üblicherweise 10 bis 60 Gew.-%, vorzugsweise 15 bis 45 Gew.-%, bezogen auf den Feed.
   Als Katalysatoren zur chemischen Spaltung der Diurethane finden z. B. die vorgenannten, die Urethanbildung katalysierenden anorganischen und organischen Verbindungen Verwendung. Vorzugsweise werden Chloride des Zinks oder Zinns sowie Zink-, Mangan-, Eisen-, oder Cobaltoxide eingesetzt, wobei der Katalysator dem Massenstrom aus der Reinigungsstufe d) vor dessen Zuführung in die Spaltung als 0,01 bis 25 Gew.-%ige, vorzugsweise 0,05 bis 10 Gew.-%ige Lösung oder Suspension in dem Alkohol, der auch zur Urethanherstellung verwendet wird, in einer Menge von 5 bis 400 ppm, vorzugsweise 10 bis 100 ppm, zudosiert wird.
   Als Spaltvorrichtungen eigenen sich beispielsweise zylinderförmige Spaltreaktoren, wie z. B. Röhrenöfen oder vorzugsweise Verdampfer, beispielsweise Fallfilm-, Dünnschichtoder Bulkverdampfer, wie z. B. Robertverdampfer, Herbertverdampfer, caddle-typ-Verdampfer, Oskarverdampfer und Heizkerzenverdampfer.
   Prinzipiell geht es darum, die mittlere Verweilzeit der Isocyanatgruppen, die bei Deblockierung des Alkohols zwangsläufig freigesetzt werden, in der Spaltzone möglichst gering zu halten und so unerwünschte Nebenreaktionen auf ein Minimum zu beschränken.
   Bevorzugt wird die Spaltung in einer kombinierten Spalt- und Rektifizierkolonne durchgeführt, die für die Energiezufuhr im Sumpf mit einem Fallfilmverdampfer, im unteren Drittel mit einer Einrichtung zum zusätzlichen Energieeintrag bzw. zur Energierückgewinnung, im oberen Drittel mit einer Einrichtung zum Abzug von vorzugsweise Roh-Diisocyanat und am Kopf mit einem Kondensator für den Rückfluss und den Abzug von reinem Alkohol ausgestattet ist.
f) Die bei der thermischen Spaltung gebildeten Spaltprodukte, die sich vor allem aus Alkohol, Diisocyanat und partiell gespaltenen Diurethanen zusammensetzen, werden durch Rektifikation bei Temperaturen von 95 bis 260 °C, vorzugsweise 110 bis 245 °C und einem Druck von 0,5 bis 250 mbar, vorzugsweise 1 bis 100 mbar, in Alkohol und in eine rohe Diisocyanatmischung, bestehend aus cycloaliphatischem Diisocyanat, partiell gespaltenem cycloaliphatischem Diisocyanat und gegebenenfalls geringen Anteilen an cycloaliphatischen Diurethan, getrennt. Diese Trennung kann beispielsweise in der Spaltkolonne der obengenannten kombinierten Spalt- und Rektifizierkolonne durchgeführt werden.
g) Die vorzugsweise durch Rektifikation erhaltene rohe Mischung bestehend aus cycloaliphatischem Diisocyanat, partiell gespaltenem cycloaliphatischem Diurethan und gegebenenfalls geringen Anteilen an cycloaliphatischen Diurethan, wird durch Destillation bei einer Temperatur von 95 bis 260 °C, vorzugsweise 110 bis 245 °C und unter einem Druck von 0,5 bis 150 mbar, vorzugsweise 1 bis 75 mbar, gereinigt, wobei die anfallenden Fraktionen zurückgeführt oder als Reinprodukt isoliert werden.
h) Die Sumpfausschleusung aus der Deblockierungsstufe e) wird mit dem Alkohol aus der Rektifikationsstufe f) zusammengeführt, wobei das Molverhältnis von NCO-Gruppen und OH-Gruppen bis zu 1 : 100, vorzugsweise 1 : 20 und besonders bevorzugt 1 : 10 beträgt, und die Reaktionsmischung in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt. Die Umsetzung kann in einer kontinuierlichen Kesselkaskade oder in einem Rohrreaktor durchgeführt werden. Als Katalysatoren kommen grundsätzlich alle Kontakte in Frage, die die NCO/OH-Reaktion fördern. Beispielhaft seien Zinnoctoat, Dibutylzinnlaurat, Zinndichlorid, Zinkdichlorid und Triethylamin aufgeführt.
i) Die Reurethanisierungsreaktion h) kann unter den bereits dargelegten Bedingungen auch in Gegenwart spezieller Katalysatoren, ausgewählt aus den Halogeniden von Fe(III) und/oder Cu(I), durchgeführt werden.
j) Ein Teil der Sumpffraktion der Reindestillation g) wird kontinuierlich ausgeschleust und optional in die Deblockierungsstufe e) oder in die Urethanisierungsstufe h) zurückgeführt. Die Rückführung in die Urethanisierungsstufe ist bevorzugt. Die Menge der Ausschleusung beträgt 0,1 bis 50 Gew.-%, vorzugsweise 0,2 bis 25 Gew.-% des Zulaufs an rohem Diisocyanat in die Reindestillationsstufe.
k) Die Kopffraktion der Reindestillationstufe g) kann vorworfen oder vorzugsweise in die Urethanisierungsstufe h) zurückgeführt werden. Die Menge der pro Zeiteinheit abgeführten Kopffraktion beträgt 0,1 bis 3 Gew.-%, vorzugsweise 0,3 bis 1 Gew.-% des Zulaufs an rohem Diisocyanat in die Reindestillation.
l) Der Stoffstrom aus der Urethanisierungsstufe g) wird in die Leicht- und Mittelsiederabtrennung c) zurückgeführt.

Mit dem erfindungsgemäßen mehrstufigen Verfahren zur kontinuierlichen Herstellung von cycloaliphatischen Diisocyanaten unter Rückführung und Ausschleusung der Nebenprodukte kann für destillierbare cycloaliphatische Diisocyanate eine mit hoher Selektivität störungsfrei ablaufende Reaktion gewährleistet werden. Das erfindungsgemäße Verfahren eignet sich insbesondere zur Herstellung von cycloaliphatischen Diisocyanaten mit 4 bis 18, vorzugsweise 5 bis 15 Kohlenstoffatomen wie 1,4-Diisocyanatocyclohexan, 4,4'-Methylendicyclohexyldiisocyanat (4,4'-H₁₂MDI), 2,2'-Methylendicyclohexyldiisocyanat (2,2'-H₁₂MDI), 2,4'-Methylendi-cyclohexyldiisocyanat (2,4'-H₁₂MDI) oder auch Mischungen der vorgenannten isomeren Methylendicyclohexyldiisocyanate (H₁₂MDI), wie sie zum Beispiel naturgemäß bei der Umwandlung von perhydriertem MDA in H₁₂MDI anfallen. Ganz besonders bevorzugt werden 4,4'-Methylendicyclohexyldiisocyanat sowie beliebige Mischungen aus 4,4'-H₁₂MDI, 2,4-H₁₂MDI und 2,2'-H₁₂MDI hergestellt.

Die hergestellten cycloaliphatischen Diisocyanate eigenen sich bestens zur Herstellung von Urethan-, Isocyanurat-, Amid- und/oder Harnstoffgruppen enthaltenden Kunststoffen nach dem Polyisocyanat-Polyadditionsverfahren. Sie finden darüber hinaus Verwendung zur Herstellung von mit Urethan-, Biuret-, und/oder Isocyanuratgruppen modifizierten Polyisocyanatmischungen. Derartige Polyisocyanatmischungen aus cycloaliphatischen Diisocyanaten werden insbesondere zur Herstellung von hochwertigen, lichtbeständigen Polyurethanbeschichtungen eingesetzt.

### Beispiele

### Beispiel 1: Erfindungsgemäße Herstellung von Methylendicyclohexyldiisocyanat (H₁₂MDI) aus perhydriertem Methylendiphenyldiamin und Harnstoff in Gegenwart von n-Butanol.

Der oberste Boden eines Destillationsreaktors wurden stündlich mit 263,0 g H₁₂MDA, 154,5 g Harnstoff und 555,9 g n-Butanol beaufschlagt und die Reaktionsmischung unter kontinuierlicher Entfernung des freigesetzten Ammoniaks bei Normalkdruck, 135 °C und einer mittleren Verweilzeit von 8 Stunden gekocht. Die im Sumpf des Destillationsreaktors anfallende Lösung von Bisharnstoff in Butanol wurde über einen Wärmetauscher auf 190 °C vorgeheizt, auf den obersten Boden eines Druckdestillationsreaktors geführt und bei 11 bis 14 bar, 220 °C und mit einer mittleren Verweilzeit von 10,5 h weiter umgesetzt. Im Sumpf des Druckdestillationsreaktors wurden stündlich 506,8 g n-Butanol eingespeist und die zusammen mit dem freigesetzten Ammoniak am Kopf abgezogene Alkoholmenge so gewählt, dass sie dem Alkoholeintrag im Sumpf entsprach. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugefährt. Die verbleibenden 628,7 g/h Bis-(4-butoxycarbonylamino-cyclohexyl)-methan (H₁₂MDU) wurden als Schmelze (140 °C) in die Wälzung des Fallfilmverdampfers der Spalt- und Rektifizierkolonne gefahren, wobei die Deblockierungsreaktion bei einer Temperatur von 237 °C und einem Sumpfdruck von 10 mbar in Gegenwart einer stationären Konzentration an Zinndichlorid von 18 ppm durchgeführt wurde. Die Spaltgase H₁₂MDI und Butanol wurden in zwei hintereinandergeschalteten Kondensatoren bei 85 °C und -25 °C auskondensiert. Das gewonnene, etwa 97 %ige Roh- H₁₂MDI wurde einer Reindestillation zugefährt, wobei 281,16 g/h H₁₂MDI mit einer Reinheit von > 99,5 % erhalten wurden, was einer auf das Amin bezogenen Ausbeute von 86 % entspricht. 183,2 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 156,1 g/h aus dem Wälzkreislauf ausgeschleust und mit 22,2 g/h Sumpfauskreisung aus der H₁₂MDI-Reindestillation sowie dem Kopfprodukt aus der Spalt- und Rektifizierkolonne vereinigt und reurethanisiert. Das Reurethanisat wurde zusammen mit dem Reaktoraustrag der Diurethanherstellung dem Flash-Behälter zugeführt.

### Beispiel 2: Erfindungsgemäße Herstellung von Methylendicyclohexyldiisocyanat (H₁₂MDI) aus perhydriertem Methylendiphenyldiamin und Harnstoff in Gegenwart von n-Butanol ― Reurethanisierung in Gegenwart von CuCl und Rückführung des Reurethanisats in die Leichtund Mittelsiederabtrennung.

Der oberste Boden eines Destillationsreaktors wurden stündlich mit 263,1 g H₁₂MDA, 154,5 g Harnstoff und 555,1 g n-Butanol beaufschlagt und die Reaktionsmischung unter kontinuierlicher Entfernung des freigesetzten Ammoniaks bei Normalkdruck, 135 °C und einer mittleren Verweilzeit von 8 Stunden gekocht. Die im Sumpf des Destillationsreaktors anfallende Lösung von Bisharnstoff in Butanol wurde über einen Wärmetauscher auf 190 °C vorgeheizt, auf den obersten Boden eines Druckdestillationsreaktors geführt und bei 11 bis 14 bar, 220 °C und mit einer mittleren Verweilzeit von 10,5 h weiter umgesetzt. Im Sumpf des Druckdestillationsreaktors wurden stündlich 510,3 g n-Butanol eingespeist und die zusammen mit dem freigesetzten Ammoniak am Kopf abgezogene Alkoholmenge so gewählt, dass sie dem Alkoholeintrag im Sumpf entsprach. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 220 °C und 2 mbar zugeführt. Die verbleibenden 631,5 g/h Bis-(4-butoxycarbonylamino-cyclohexyl)-methan (H₁₂MDU) wurden als Schmelze (140 °C) in die Wälzung des Fallfilmverdampfers der Spalt- und Rektifizierkolonne gefahren, wobei die Deblockierungsreaktion bei einer Temperatur von 235 °C und einem Sumpfdruck von 10 mbar in Gegenwart einer stationären Konzentration an Zinndichlorid von 15 ppm durchgeführt wurde. Die Spaltgase H₁₂MDI und Butanol wurden in zwei hintereinandergeschalteten Kondensatoren bei 85 °C und -25°C auskondensiert. Das gewonnene, etwa 97 %ige Roh- H₁₂MDI wurde einer Reindestillation zugefährt, wobei 284,28 g/h H₁₂MDI mit einer Reinheit von > 99,5 % erhalten wurden, was einer auf das Amin bezogenen Ausbeute von 87 % entspricht. 186,3 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 154,8 g/h aus dem Wälzkreislauf ausgeschleust und mit 21,9 g/h Sumpfauskreisung aus der H₁₂MDI-Reindestillation sowie dem Kopfprodukt aus der Spalt- und Rektifizierkolonne vereinigt und in Gegenwart von 100 ppm CuCl reurethanisiert. Das Reurethanisat wurde zusammen mit dem Reaktoraustrag der Diurethanherstellung dem Flash-Behälter zugefährt.

## Patentansprüche

1. Mehrstufiges Verfahren zur kontinuierlichen Herstellung von cycloaliphatischen Diisocyanaten, durch Umsetzung von cycloalipahtischen Diaminen mit Kohlensäurederivaten und Alkoholen zu cycloaliphatischen Diurethanen und anschließenden thermischen Spaltung der Diurethane zu cycloaliphatischen Diisocyanten,
**dadurch gekennzeichnet,**
**dass** die Bildung der Diurethane zweistufig ausgeführt ist, das von Leicht-, Mittel- und Hochsiedern befreite Diurethan unter Freisetzung des gewünschten Düoscyanats thermisch gespalten wird, ein Teil des Spaltsumpfes der Spaltapparatur kontiniuerlich ausgeschleust und mit Alkohol reurethanisiert wird und die Rückführung des Reurethanisatstroms direkt in die Leichtsiederabtrennung erfolgt.

2. Mehrstufiges Verfahren zur kontinuierlichen Herstellung von cycloaliphatischen Diisocyanaten der Formel (I)
OCN-R-NCO
wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Kohlensäurederivaten und Alkoholen zu Diurethanen, und deren thermische Spaltung, wobei
a) cycloaliphatische Diamine der Formel (II)
H₂N-R-NH₂
wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)
R¹-OH
wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Katalysatoren zu Cycloalkylenbisharnstoffen der Formel (IV)
H₂N-OC-HN-R-NH-CO-NH₂
wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;
b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)
R¹O-OC-HN-R-NH-CO-OR¹
überführt wird;
c) aus der erhaltenen Reaktionsmischung der Alkohol, die Dialkylcarbonate und/oder Carbamidsäurealkylester abgetrennt und der Alkohol in die Reaktionsstufe a) zurückführt wird;
d) der Stoffstrom aus Stufe c) destillativ in einen Wertstoffstrom und einen Nebenproduktstrom, welcher ausgeschleust wird, getrennt wird;
e) die über die Schritte c) und d) aufgereinigte Diurethane enthaltende Reaktionsmischung in Gegenwart eines Katalysators lösemittelfrei bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig ausgeschleust wird;
f) die Spaltprodukte durch Rektifikation in ein rohes Diisocyanat und Alkohol getrennt werden;
g) das rohe cycloaliphatische Diisocyanat durch Destillation gereinigt und die Reinproduktfraktion isoliert wird;
h) die Sumpfausschleusung aus e) mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;
i) optional die Reurethanisierungsreaktion h) in Gegenwart spezieller Katalysatoren, ausgewählt aus den Halogeniden von Fe(III) und/oder Cu(I), durchgeführt wird;
j) ein Teil der Sumpffraktion der Reindestillation g) kontinuierlich ausgeschleust und in die Spaltreaktion e), und/oder in die Urethanisierungsstufe h) geführt wird;
k) optional die bei der Reindestillation des rohen cycloaliphatischen Diisocyanats anfallende Kopffraktion ebenfalls in die Urethanisierungsstufe h) zurückgeführt wird;
l) der Reurethanisatstrom aus h) in Stufe c) zurückgeführt wird.

3. Mehrstufiges Verfahren nach Anspruch 1 oder 2,
**dadurch gekennzeichnet,**
das als cycloaliphatisches Diamin 4,4'-Methylendicyclohexyldiamin, 2,4-Methylendicyclohexyldiamin und 2,2'-Methylendicyclohexyldiamin sowie auch beliebige Mischungen mindestens zweier dieser Isomere eingesetzt werden.

4. Mehrstufiges Verfahren nach Anspruch 1 oder 2,
**dadurch gekennzeichnet,**
**dass** als cycloaliphatisches Diamin 4,4'-Methylendicyclohexyldiamin und/oder isomere cycloaliphatische Diamine eingesetzt werden.

5. Mehrstufiges Verfahren nach Anspruch 1,
**dadurch gekennzeichnet,**
**dass** als cycloaliphatisches Diamin 1,4-Diaminocyclohexan eingesetzt wird.

6. Verfahren nach mindestens einem der vorherigen Ansprüche,
**dadurch gekennzeichnet,**
**dass** die Stufe a) in einem Reaktor bei 100 bis 145 °C und einem Druck von 0,7 bis 1,8 bar durchgeführt wird.

7. Verfahren nach mindestens einem der vorherigen Ansprüche,
**dadurch gekennzeichnet,**
**dass** die Stufe a) in einem Destillationsreaktor durchgeführt wird.

8. Verfahren nach mindestens einem der vorherigen Ansprüche,
**dadurch gekennzeichnet,**
**dass** die Umsetzung in Stufe a) in einem Molverhältnis von Diamin : Harnstoff: Alkohol von 1 : 2,0 bis 2,4 : 3 bis 10 erfolgt.

9. Verfahren nach mindestens einem der vorherigen Ansprüche,
wobei in Stufe a) die Edukte kontinuierlich auf den obersten Boden aufgegeben werden und das freigesetzte Ammoniak durch Alkoholbrüden, die im Sumpf des Destillationsreaktor eingeführt werden, ausgetrieben wird.

10. Verfahren nach mindestens einem der vorherigen Ansprüche,
wobei die Verweilzeit der Edukte in Stufe a) 4 bis 10, vorzugsweise 5 bis 9 Stunden beträgt.

11. Verfahren nach mindestens einem der vorherigen Ansprüche,
**dadurch gekennzeichnet,**
**dass** die Stufe b) in einem Druckdestillationsreaktor durchgeführt wird.

12. Verfahren nach mindestens einem der vorherigen Ansprüche,
**dadurch gekennzeichnet,**
**dass** in Stufe b) bei einem molaren Verhältnis von Bisharnstoff zu Alkohol von 1 : 5 bis 12 verfahren wird.

13. Verfahren nach mindestens einem der vorherigen Ansprüche,
**dadurch gekennzeichnet,**
**dass** der Stoffstrom aus a) vorzugsweise kontinuierlich auf den obersten Boden des Reaktors der Stufe b) gefahren wird.

14. Verfahren nach mindestens einem der vorherigen Ansprüche,
**dadurch gekennzeichnet,**
**dass** in Stufe b) bei Reaktionstemperaturen von 140 bis 270 °C, vorzugsweise 160 bis 250 °C und unter einem Druck, der 5 bis 20 bar, vorzugsweise 7 bis 15 bar beträgt, umgesetzt wird.

15. Verfahren nach mindestens einem der vorherigen Ansprüche,
**dadurch gekennzeichnet,**
**dass** die Umsetzung in Stufe a) und/oder in Stufe b) in Gegenwart von Katalysatoren durchgeführt wird.

16. Verfahren nach mindestens einem der vorherigen Ansprüche,
**dadurch gekennzeichnet,**
**dass** die Stufe c) zweistufig durchgeführt wird.

17. Verfahren nach Anspruch 16,
**dadurch gekennzeichnet,**
**dass** auf der ersten Stufe die Reaktionsmischung vom Druckniveau der Reaktionsstufe b) auf einen Druck von 1 bis 500 mbar, vorzugsweise 2 bis 150 mbar, entspannt wird.

18. Verfahren nach den Ansprüchen 16 oder 17,
**dadurch gekennzeichnet,**
**dass** die Brüden der Stufe c) nach weiterer destillativer Reinigung in die Reaktionsstufe a) zugeführt werden.

19. Verfahren nach mindestens einem der vorhergehenden Ansprüche,
**dadurch gekennzeichnet,**
**dass** die Trennung in Stufe d) bei einer Temperatur von 180 bis 260 °C, vorzugsweise 200 bis 240 °C und unter einem Druck von 0,01 bis 10 mbar, vorzugsweise 0,02 bis 5 mbar durchgeführt wird.

20. Verfahren nach mindestens einem der vorhergehenden Ansprüche,
**dadurch gekennzeichnet,**
**dass** die Stufe d) mit Hilfe eines Dünnschicht- oder Kurzwegverdampfers durchgeführt wird.

21. Verfahren nach mindestens einem der vorhergehenden Ansprüche,
**dadurch gekennzeichnet,**
**dass** die Nebenprodukte aus Stufe d) ausgeschleust und verworfen werden.

22. Verfahren nach mindestens einem der vorhergehenden Ansprüche,
**dadurch gekennzeichnet,**
**dass** der Stoffstrom aus Stufe c) nicht gemäß Stufe d) verarbeitet wird, sondern vor seiner destillativen Aufreinigung in zwei Teilströmen aufgeteilt wird, von denen ein Teilstrom direkt der Deblockierungsreaktion (siehe e) zugeführt wird.

23. Verfahren nach mindestens einem der vorhergehenden Ansprüche,
**dadurch gekennzeichnet,**
**dass** die Stufe e) in einer kombinierten Spalt- und Rektifizierkolonne durchgeführt wird.

24. Verfahren nach mindestens einem der vorhergehenden Ansprüche,
**dadurch gekennzeichnet,**
**dass** in Stufe e) in Gegenwart von Katalysatoren bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch gespalten wird.

25. Verfahren nach mindestens einem der vorhergehenden Ansprüche,
**dadurch gekennzeichnet,**
**dass** in Stufe e) lösemittelfrei in flüssiger Phase gespalten wird.

26. Verfahren nach mindestens einem der vorhergehenden Ansprüche,
**dadurch gekennzeichnet,**
**dass** die Stufe e) in Gegenwart von Katalysatoren durchgeführt wird.

27. Verfahren nach mindestens einem der vorhergehenden Ansprüche,
**dadurch gekennzeichnet,**
**dass** die thermisch induzierte Diurethanspaltung in Röhrenöfen oder vorzugsweise Verdampfern, beispielsweise Fallfilm-, Dünnschicht- oder Bulkverdampfern, wie z. B. Robertverdampfern, Herbertverdampfern, caddle-typ-Verdampfern, Oskarverdampfern und Heizkerzenverdampfern durchgeführt wird

28. Verfahren nach mindestens einem der vorhergehenden Ansprüche,
**dadurch gekennzeichnet,**
**dass** in Stufe e) der Umsatz von Diurethan zu Diisocyanat in Abhängigkeit vom verwendeten Diurethan frei gewählt wird, bevorzugt in einem Bereich von 10 bis 95 Gew.-%, vorzugsweise 35 bis 85 Gew.-% der zugeführten Diurethanmenge (Feed) liegt.

29. Verfahren nach mindestens einem der vorhergehenden Ansprüche,
**dadurch gekennzeichnet,**
**dass** in Stufe e) ein Teil der Reaktionsmischung, der nicht umgesetzten Diurethane, hochsiedende Nebenprodukte und andere wieder verwertbare und unverwertbare Nebenprodukte enthält, kontinuierlich ausgeschleust wird.

30. Verfahren nach Anspruch 28,
**dadurch gekennzeichnet,**
**dass** die Menge der Ausschleusung 10 bis 60 Gew.-%, vorzugsweise 15 bis 45 Gew.-%, bezogen auf den Feed, beträgt.

31. Verfahren nach mindestens einem der vorhergehenden Ansprüche,
**dadurch gekennzeichnet,**
**dass** die Stufe h) in einer kontinuierlichen Kesselkaskade oder in einem Rohrreaktor durchgeführt wird.

32. Verfahren nach mindestens einem der vorhergehenden Ansprüche,
**dadurch gekennzeichnet,**
**dass** die Umsetzung in Stufe h) in Gegenwart von Katalysatoren aus der Gruppe der Sn-und/oder Zn-Carboxylate oder -Halogenide und/oder tert. Amine erfolgt.

33. Verfahren nach mindestens einem der vorhergehenden Ansprüche,
**dadurch gekennzeichnet,**
**dass** in Stufe j) die Menge der Ausschleusung 0,1 bis 50 Gew.-%, vorzugsweise 0,2 bis 25 Gew.-% des Zulaufs an rohem Polyisocyanat in die Reindestillationsstufe beträgt.

34. Verfahren nach mindestens einem der vorhergehenden Ansprüche,
**dadurch gekennzeichnet,**
**dass** 1,4-Diisocyanatocyclohexan, 4,4'-Methylendicyclohexyldiisocyanat, 2,2'-Methylendicyclohexyldiisocyanat, 2,4'-Methylendicyclohexyldiisocyanat oder auch beliebige Mischungen mindestens zweier isomerer Methylendicyclohexyldiisocyanate hergestellt werden.

35. Verfahren nach mindestens einem der vorherigen Ansprüche,
**dadurch gekennzeichnet,**
**dass** Diamine ausgewählt aus 1,3- und 1,4-Diaminomethylcyclohexan und 3-Aminomethyl-3,5,5-trimethylcyclohexylamin eingesetzt werden.

## Claims

1. Multistage process for continuously preparing cycloaliphatic diisocyanates, by reacting cycloaliphatic diamines with carbonic acid derivatives and alcohols to give cycloaliphatic bisureas and subsequently thermally cleaving the bisureas to give cycloaliphatic diisocyanates,
**characterized in that**
the formation of the diurethanes is performed in two stages, the diurethane freed of low, medium and high boilers is thermally cleaved to release the desired diisocyanate, a portion of the cleavage residue from the cleavage apparatus is continuously discharged and reurethanized with alcohol and the reurethanization product is recycled directly into the low-boiler separation.

2. Multistage process for continuously preparing cycloaliphatic diisocyanates of the formula (I)
OCN-R-NCO
where R is a bivalent cycloaliphatic hydrocarbon radical having from 4 to 18, preferably from 5 to 15, carbon atoms, with the proviso that the two isocyanate groups are bonded directly to a hydrocarbon cycle and at least 3 carbon atoms are disposed between them, by reacting cycloaliphatic diamines with carbonic acid derivatives and alcohols to give diurethanes and thermally cleaving them, wherein
a) cycloaliphatic diamines of the formula (II)
H₂N-R-NH₂
where R is a bivalent cycloaliphatic hydrocarbon radical having from 4 to 18, preferably from 5 to 15, carbon atoms, where the two nitrogen atoms are bonded directly to at least one hydrocarbon cycle and at least 3 carbon atoms are disposed between them, are reacted with urea and in the presence of alcohols of the formula (III)
R¹-OH
where R¹ is a radical as remains after removal of the hydroxyl group from a primary or secondary (cyclo)aliphatic alcohol having from 3 to 8 carbon atoms, in the absence or presence of catalysts, to give cycloalkylenebisureas of the formula (IV);
H₂N-OC-HN-R-NH-CO-NH₂
where R is a bivalent cycloaliphatic hydrocarbon radical having from 4 to 18, preferably from 5 to 15, carbon atoms, with the proviso that the two nitrogen atoms adjacent to R are bonded directly to a hydrocarbon cycle and at least 3 carbon atoms are disposed between them, and the ammonia formed is simultaneously removed continuously;
b) the resulting crude cycloalkylenebisurea is converted in a second reactor using the alcohol of the formula (III) used as a solvent while continuously driving out the ammonia released to cycloalkylenediurethane of the formula (V)
R¹O-OC-HN-R-NH-CO-OR¹;
c) the alcohol, the dialkyl carbonates and/or alkyl carbamates are removed from the resulting reaction mixture, and the alcohol is recycled into the reaction stage a);
d) the material stream from stage c) is separated by distillation into a material-of-value stream and a by-product stream which is discharged;
e) the reaction mixture comprising the diurethanes purified by steps c) and d) is continuously and thermally cleaved in the presence of a catalyst and without solvent, at temperatures of from 180 to 280°C, preferably from 200 to 260°C, and under a pressure of from 0.1 to 200 mbar, preferably from 0.2 to 100 mbar, in such a way that a portion of the reaction mixture of from 10 to 60% by weight based on the feed, preferably from 15 to 45% by weight based on the feed, is constantly discharged;
f) the cleavage products are separated by rectification into a crude diisocyanate and alcohol;
g) the crude cycloaliphatic diisocyanate is purified by distillation, and the pure product fraction is isolated;
h) the bottoms discharge from e) is reacted with the alcohol from f) in the presence or absence of catalysts within from 1 to 150 min, preferably from 3 to 60 min, at temperatures of from 20 to 200°C, preferably from 50 to 170°C, and a pressure of from 0.5 to 20 bar, preferably from 1 to 15 bar, at a molar ratio of NCO groups to OH groups of up to 1 : 100, preferably 1 : 20 and more preferably 1 : 10;
i) the reurethanization reaction h) is optionally carried out in the presence of specific catalysts selected from the halides of Fe(III) and/or Cu(I);
j) a portion of the bottoms fraction of the purification by distillation g) is continuously discharged and conducted into the cleavage reaction e) and/or into the urethanization stage h);
k) optionally, the top fraction obtained in the purification by distillation of the crude cycloaliphatic diisocyanate is likewise recycled into the urethanization stage h);
l) the reurethanized stream from h) is recycled into stage c).

3. Multistage process according to Claim 1 or 2,
**characterized in that,**
the cycloaliphatic diamine used is 4,4'-dicyclohexylmethanediamine, 2,4-dicyclohexylmethanediamine and 2,2'-dicyclohexylmethanediamine, and also any mixtures of at least two of these isomers.

4. Multistage process according to Claim 1 or 2,
**characterized in that,**
the cycloaliphatic diamine used is 4,4'-dicyclohexylmethanediamine and/or isomeric cycloaliphatic diamines.

5. Multistage process according to Claim 1,
**characterized in that,**
the cycloaliphatic diamine used is 1,4-diaminocyclohexane.

6. Process according to at least one of the preceding claims,
**characterized in that,**
stage a) is carried out in a reactor at from 100 to 145°C and a pressure of from 0.7 to 1.8 bar.

7. Process according to at least one of the preceding claims,
**characterized in that,**
stage a) is carried out in a distillation reactor.

8. Process according to at least one of the preceding claims,
**characterized in that,**
the reaction in stage a) is effected in a molar ratio of diamine : urea : alcohol of from 1 : 2.0 to 2.4 : 3 to 10.

9. Process according to at least one of the preceding claims,
**characterized in that,** in stage a), the reactants are supplied continuously to the uppermost tray and the ammonia released is driven out by alcohol vapors which are introduced into the bottom of the distillation reactor.

10. Process according to at least one of the preceding claims,
wherein the residence time of the reactants in stage a) is from 4 to 10 hours, preferably from 5 to 9 hours.

11. Process according to at least one of the preceding claims,
**characterized in that,**
stage b) is carried out in a pressure distillation reactor.

12. Process according to at least one of the preceding claims,
**characterized in that,**
stage b) is conducted at a molar ratio of bisurea to alcohol of from 1 : 5 to 12.

13. Process according to at least one of the preceding claims,
**characterized in that,**
the stream from a) is conducted preferably continuously to the uppermost tray of the reactor of stage b).

14. Process according to at least one of the preceding claims,
**characterized in that,**
conversion is effected in stage b) at reaction temperatures of from 40 to 270°C, preferably from 160 to 250°C, and under a pressure of from 5 to 20 bar, preferably from 7 to 15 bar.

15. Process according to at least one of the preceding claims,
**characterized in that,**
the reaction in stage a) and/or in stage b) is carried out in the presence of catalysts.

16. Process according to at least one of the preceding claims,
**characterized in that,**
stage c) is carried out in two stages.

17. Process according to Claim 16,
**characterized in that,**
at the first stage, the reaction mixture is decompressed from the pressure level of reaction stage b) to a pressure of from 1 to 500 mbar, preferably from 2 to 150 mbar.

18. Process according to Claims 16 and 17,
**characterized in that**,
the vapors of stage c) are fed, after further distillative purification, into reaction stage a).

19. Process according to at least one of the preceding claims,
**characterized in that,**
the separation in stage d) is carried out at a temperature of from 180 to 260°C, preferably from 200 to 240°C, and under a pressure of from 0.01 to 10 mbar, preferably from 0.02 to 5 mbar.

20. Process according to at least one of the preceding claims,
**characterized in that,**
stage d) is carried out with the aid of a thin-film or short-path evaporator.

21. Process according to at least one of the preceding claims,
**characterized in that,**
the by-products from stage d) are discharged and discarded.

22. Process according to at least one of the preceding claims,
**characterized in that,**
the stream in stage c) is not processed as per stage d), but rather is divided before its distillative purification into two substreams of which one substream is fed directly to the deblocking reaction (see e).

23. Process according to at least one of the preceding claims,
**characterized in that,**
stage e) is carried out in a combined cleavage and rectification column.

24. Process according to at least one of the preceding claims,
**characterized in that,**
in stage e), in the presence of catalysts, thermal cleavage is effected continuously at temperatures of from 180 to 280°C, preferably from 200 to 260°C, and under a pressure of from 0.1 to 200 mbar, preferably from 0.2 to 100 mbar.

25. Process according to at least one of the preceding claims,
**characterized in that,**
in stage e), cleavage is effected without solvent in the liquid phase.

26. Process according to at least one of the preceding claims,
**characterized in that**,
stage e) is carried out in the presence of catalysts.

27. Process according to at least one of the preceding claims,
**characterized in that,**
the thermally induced diurethane cleavage is carried out in tubular furnaces or preferably evaporators, for example falling-film, thin-film or bulk evaporators, such as Robert evaporators, Herbert evaporators, Caddle-type evaporators, Oskar evaporators and heating cartridge evaporators.

28. Process according to at least one of the preceding claims,
**characterized in that,**
in stage e), the conversion of diurethane to diisocyanate is selected freely depending on the diurethane used, preferably within the range of from 10 to 95% by weight, preferably from 35 to 85% of the diurethane feed.

29. Process according to at least one of the preceding claims,
**characterized in that,**
in stage e), a portion of the reaction mixture which comprises unconverted diurethanes, high-boiling by-products and other reutilizable and nonutilizable by-products is continuously discharged.

30. Process according to Claim 30,
**characterized in that,**
the amount of the discharge is from 10 to 60% by weight, preferably from 15 to 45% by weight, based on the feed.

31. Process according to at least one of the preceding claims,
**characterized in that,**
stage h) is carried out in a continuous tank battery or in a tubular reactor.

32. Process according to at least one of the preceding claims,
**characterized in that,**
the reaction in stage h) is effected in the presence of catalysts from the group of tin and/or zinc carboxylates or halides and/or tertiary amines.

33. Process according to at least one of the preceding claims,
**characterized in that,**
in stage j), the amount of the discharge is from 0.1 to 50% by weight, preferably from 0.2 to 25% by weight, of the feed of crude polyisocyanate into the purifying distillation.

34. Process according to at least one of the preceding claims,
**characterized in that,**
1,4-diisocyanatocyclohexane, 4,4'-dicyclohexylmethane diisocyanate, 2,2'-dicyclohexylmethane diisocyanate, 2,4'-dicyclohexylmethane diisocyanate or else any mixtures of at least two isomeric dicyclohexylmethane diisocyanates are prepared.

35. Process according to at least one of the preceding claims,
**characterized in that,**
diamines selected from 1,3- and 1,4-diaminomethylcyclohexane and 3-aminomethyl-3,5,5-trimethylcyclohexylamine are used.

## Revendications

1. Procédé à plusieurs étapes en vue de la fabrication en continu de diisocyanates cycloaliphatiques, par réaction de diamines cycloaliphatiques avec des dérivés de l'acide carbonique et d'alcools pour former des diuréthannes cycloaliphatiques et par clivage thermique subséquent des diuréthannes pour fermer des diisocyanates cycloaliphatiques, **caractérisé en ce que** la formation des diuréthannes est exécutée en deux étapes, **en ce que** le diuréthane libéré des produits à point d'ébullition faible, moyen et élevé est clivé par voie thermique avec dégagement du diisocyanate souhaité, **en ce qu'**une partie du puits de clivage de l'appareil de clivage est évacuée en continu et subit une re-uréthanisation à l'aide d'alcool et **en ce que** la reconduite du courant de re-uréthanisation se fait directement dans la séparation des produits à point d'ébullition faible.

2. Procédé en vue de la fabrication en continu de diisocyanates cycloaliphatiques de la formule (I)
OCN-R-NCO
R désignant un radical hydrocarboné cycloaliphatique divalent ayant de 4 à 18, de préférence de 5 à 15 atomes de carbone, sous réserve que les deux groupements isocyanate soient directement liés à un cycle hydrocarboné et qu'au moins 3 atomes de carbone soient disposés entre eux, par la réaction de diamines cycloaliphatiques avec des dérivés de l'acide carbonique et d'alcools pour former des diuréthannes et par leur clivage thermique
a) des diamines cycloaliphatiques de la formule (II)
H₂N-R-NH₂
R désignant un radical hydrocarboné cycloaliphatique divalent ayant de 4 à 18, de préférence de 5 à 15 atomes de carbone, les deux atomes d'azote étant liés directement à au moins un cycle hydrocarboné et au moins 3 atomes de carbone étant disposés entre eux, réagissant avec l'urée et en présence d'alcools de la formule (III)
R¹-OH
R¹ désignant un radical, comme il est obtenu après élimination du groupement hydroxyle d'un alcool (cyclo)aliphatique primaire ou secondaire, ayant de 3 à 8 atomes de carbone, en l'absence ou en présence de catalyseurs, pour former des cycloalcylène-bis-urées de la formule (IV)
H₂N-OC-HN-R-NH-CO-NH₂
R désignant un radical hydrocarboné cycloaliphatique divalent ayant de 4 à 18, de préférence de 5 à 15 atomes de carbone, sous réserve que les deux atomes d'azote flanquant les R soient liés directement à un cycle hydrocarboné et qu'au moins 3 atomes de carbone soient disposés entre eux, et l'ammoniac qui se forme étant simultanément éliminé en continu;
b) le cycloalcylène-bis-urée brut se formant étant converti dans un deuxième réacteur avec l'alcool de la formule (III), utilisé en tant que solvant dans a), sous explusion en continu de l'ammoniac libéré en cycloalcylène-diuréthanne de la formule (V)
R¹O-OC-HN-R-NH-CO-OR¹
c) les carbonates dialkyle et/ou les esters alkyle de l'acide carbamide étant éliminés du mélange réactionnel obtenu et l'alcool étant reconduit à l'étape de réaction a);
(d) le courant de substances provenant de l'étape c) étant séparé par distillation en un courant de substances de valeur et en un courant de produits secondaires, qui est évacué;
(e) le mélange réactionnel contenant les diuréthannes purifiés par l'intermédiaire des étapes c) et d) étant clivé en continu par voie thermique en présence d'un catalyseur, en l'absence de solvants, à des températures de 180 à 280 °C, de préférence de 200 à 260 °C, et à une pression de 0,1 à 200 mbars, de préférence de 0,2 à 100 mbars, de telle sorte qu'une partie du mélange réactionnel de 10 à 60 % en poids, par rapport à l'alimentation, de préférence de 15 à 45 % en poids, soit constamment évacuée;
f) les produits de clivage étant séparés par rectification en un diisocyanate brut et en un alcool;
g) le diisocyanate cycloaliphatique brut étant purifié par distillation et la fraction de produit pur étant isolée;
h) l'évacuation du puits provenant de e) réagissant avec l'alcool provenant de f) en présence ou en l'absence de catalyseurs au cours d'une période de 1 à 150 minutes, de préférence de 3 à 60 minutes, à des températures de 20 à 200 °C, de préférence de 50 à 170 °C et à une pression de 0,5 à 20 bars, de préférence de 1 à 15 bars, et le rapport molaire des groupements NCO et des groupements OH étant de jusqu'à 1 : 100, de préférence 1 : 20 et, en particulier, de préférence 1 : 10;
i) la réaction de re-uréthanisation h) étant effectuée en option en présence de catalyseurs spéciaux, sélectionnés parmi les halogénures de Fe (III) et/ou de Cu(I);
j) une partie de la fraction de puits de la distillation pure g) étant évacuée en continu et étant conduite dans la réaction de clivage e), et/ou dans l'étape d'uréthanisation h);
k) la fraction de tête se formant lors de la distillation pure du diisocyanate cycloaliphatique brut étant en option reconduite également à l'étape d'uréthanisation h);
l) le courant de re-uréthanisation provenant de h) étant reconduit dans l'étape c).

3. Procédé à plusieurs étapes selon la revendication 1 ou 2, **caractérisé en ce que** l'on utilise, en tant que diamine cycloaliphatique, la 4,4'-méthylènedicyclohexyldiamine, la 2,4-méthylènedicyclohexyldiamine et la 2,2'-méthylènedicyclohexyldiamine ainsi qu'également des mélanges quelconques d'au moins deux de ces isomères.

4. Procédé à plusieurs étapes selon la revendication 1 ou 2, **caractérisé en ce que** l'on utilise, en tant que diamine cycloaliphatique, la 4,4'-méthylènedicyclohexyldiamine et/ou des diamines cycloaliphatiques isomères.

5. Procédé à plusieurs étapes selon la revendication 1, **caractérisé en ce que** l'on utilise en tant que diamine cycloaliphatique le 1,4-diaminocyclohexane.

6. Procédé selon au moins l'une quelconque des revendications précédentes, **caractérisé en ce que** l'étape a) est effectuée dans un réacteur à la température de 100 à 145 °C et à une pression de 0,7 à 1,8 bar.

7. Procédé selon au moins l'une quelconque des revendications précédentes, **caractérisé en ce que** l'étape a) est effectuée dans un réacteur de distillation.

8. Procédé selon au moins l'une quelconque des revendications précédentes, **caractérisé en ce que** la réaction dans l'étape a) se fait dans un rapport molaire diamine:urée:alcool allant de 1:2,0 à 2,4:3 à 10.

9. Procédé selon au moins l'une quelconque des revendications précédentes, les produits de départ, dans l'étape a), étant libérés en continu sur le plateau le plus élevé et l'ammoniac libéré étant expulsé par des vapeurs d'alcool, qui sont introduites dans le puits du réacteur de distillation.

10. Procédé selon au moins l'une quelconque des revendications précédentes, la durée de séjour des produits de départ dans l'étape a) étant de 4 à 10, de préférence de 5 à 9 heures.

11. Procédé selon au moins l'une quelconque des revendications précédentes, **caractérisé en ce que** l'étape b) est effectuée dans un réacteur de distillation sous pression.

12. Procédé selon au moins l'une quelconque des revendications précédentes, **caractérisé en ce que** dans l'étape b), on opère avec un rapport molaire de bis-urée à alcool de 1:5 à 12.

13. Procédé selon au moins l'une quelconque des revendications précédentes, **caractérisé en ce que** le courant de substances de a) est amené de préférence en continu sur le plateau le plus élevé du réacteur de l'étape b).

14. Procédé selon au moins l'une quelconque des revendications précédentes, **caractérisé en ce que** dans l'étape b), on effectue la réaction à des températures de réaction de 140 à 270°C, de préférence de 160 à 250°C et à une pression qui est de 5 à 20 bars, de préférence de 7 à 15 bars.

15. Procédé selon au moins l'une quelconque des revendications précédentes, **caractérisé en ce que** la réaction dans l'étape a) et/ou dans l'étape b) est effectuée en présence de catalyseurs.

16. Procédé selon au moins l'une quelconque des revendications précédentes, **caractérisé en ce que** l'étape c) est effectuée en deux étapes.

17. Procédé selon la revendication 16, **caractérisé en ce que** sur la première étape le mélange réactionnel est détendu du niveau de pression de l'étape de réaction b) à une pression de 1 à 500 mbars, de préférence, de 2 à 150 mbars.

18. Procédé selon les revendications 16 ou 17, **caractérisé en ce que** les vapeurs de l'étape c), après purification par distillation supplémentaire, sont acheminées à l'étape de réaction a).

19. Procédé selon au moins l'une quelconque des revendications précédentes, **caractérisé en ce que** la séparation dans l'étape d) est effectuée à une température de 180 à 260°C, de préférence de 200 à 240°C et à une pression de 0,01 à 10 mbars, de préférence de 0,02 à 5 mbars.

20. Procédé selon au moins l'une quelconque des revendications précédentes, **caractérisé en ce que** l'étape d) est effectuée à l'aide d'un évaporateur à couches minces ou d'un évaporateur moléculaire.

21. Procédé selon au moins l'une quelconque des revendications précédentes, **caractérisé en ce que** les produits secondaires sont évacués hors de l'étape d) et sont mis au rebut.

22. Procédé selon au moins l'une quelconque des revendications précédentes, **caractérisé en ce que** le courant de substances de l'étape c) n'est pas traité conformément à l'étape d), mais est subdivisé, avant sa purification par distillation, en deux courants partiels, dont un courant partiel est acheminé directement à la réaction de déblocage (voir e).

23. Procédé selon au moins l'une quelconque des revendications précédentes, **caractérisé en ce que** l'étape e) est effectuée dans une colonne de clivage et de rectification combinée.

24. Procédé selon au moins l'une quelconque des revendications précédentes, **caractérisé en ce que**, dans l'étape e), en présence de catalyseurs à des températures de 180 à 280 °C, de préférence de 200 à 260 °C, et à une pression de 0,1 à 200 mbars, de préférence de 0,2 à 100 mbars, on procède en continu au clivage thermique.

25. Procédé selon au moins l'une quelconque des revendications précédentes, **caractérisé en ce que,** dans l'étape e), on procède au clivage sans solvants en phase liquide.

26. Procédé selon au moins l'une quelconque des revendications précédentes, **caractérisé en ce que** l'étape e) est effectuée en présence de catalyseurs.

27. Procédé selon au moins l'une quelconque des revendications précédentes, **caractérisé en ce que** le clivage de diuréthannes induit par voie thermique est effectué dans des fours tubulaires ou, de préférence dans des évaporateurs, par exemple dans des évaporateurs à film descendant, à couches minces ou en vrac, comme, par exemple des évaporateurs Robert, des évaporateurs Herbert, des évaporateurs dits de type caddle, des évaporateurs Oskar et des évaporateurs à bougies de chauffage.

28. Procédé selon au moins l'une quelconque des revendications précédentes, **caractérisé en ce que**, dans l'étape e), la conversion du diuréthanne en diisocyanate est choisie librement en fonction du diuréthanne utilisé, de préférence, dans une plage de 10 à 95 % en poids, de préférence de 35 à 85 % en poids de la quantité de diuréthanne acheminée (alimentation).

29. Procédé selon au moins l'une quelconque des revendications précédentes, **caractérisé en ce que** dans l'étape e) une partie du mélange réactionnel, qui contient des diuréthannes n'ayant pas réagi, des produits secondaires à point d'ébullition élevé et d'autres produits secondaires récupérables et non capables de récupération, est évacuée en continu.

30. Procédé selon au moins la revendication 28, **caractérisé en ce que** la quantité de l'évacuation est de 10 à 60 % en poids, de préférence de 15 à 45 % en poids, par rapport à l'alimentation.

31. Procédé selon au moins l'une quelconque des revendications précédentes, **caractérisé en ce que** l'étape h) est effectuée dans une cascade continue de récipients ou dans un réacteur tubulaire.

32. Procédé selon au moins l'une quelconque des revendications précédentes, **caractérisé en ce que** la réaction dans l'étape h) se fait en présence de catalyseurs choisis parmi le groupe des carboxylates ou des halogénures de Sn et/ou de Zn et/ou des amines tertiaires.

33. Procédé selon au moins l'une quelconque des revendications précédentes, **caractérisé en ce que** dans l'étape j), la quantité de l'évacuation est de 0,1 à 50 % en poids, de préférence de 0,2 à 25 % en poids de l'apport en polyisocyanate brut dans l'étape de distillation pure.

34. Procédé selon au moins l'une quelconque des revendications précédentes, **caractérisé en ce que** l'on fabrique le 1,4-diisocyanatocyclohexane, le diisocyanate de 4,4'-méthylènedicyclohexyle, le diisocyanate de 2,2'-méthylènedicyclohexyle, le diisocyanate de 2,4'-méthylènedicyclohexyle ou aussi des mélanges quelconques d'au moins deux diisocyanates de méthylènedicyclohexyle isomères.

35. Procédé selon au moins l'une quelconque des revendications précédentes, **caractérisé en ce que** l'on utilise des diamines sélectionnées parmi les 1,3- et 1,4-diaminométhylcyclohexanes et la 3-aminométhyl-3,5,5-triméthylcyclohexylamine.
